# EUROPEAN PATENT APPLICATION

(11) **EP 1 637 071 A1**
(43) Date of publication of application: **22.03.2006**
(21) Application number: 05019793.8
(22) Date of filing: 12.09.2005
(51) Int. Cl.: A61B 5/022

(54) **Cuff for blood pressure monitor**

(30) Priority: 15.09.2004 JP 2004268515
(71) Applicant: Omron Healthcare Co., Ltd., Kyoto 615-0084 (JP)
(72) Inventor: Kishimoto, Hiroshi Omron Healthcare Co., Ltd., Ukyo-ku Kyoto-shi Kyoto 615-0084 (JP); Sano, Yoshihiko Omron Healthcare Co., Ltd., Ukyo-ku Kyoto-shi Kyoto 615-0084 (JP); Karo, Hiromichi Omron Healthcare Co., Ltd., Ukyo-ku Kyoto-shi Kyoto 615-0084 (JP)
(74) Representative: Kilian, Helmut

(57) **Abstract**

A cuff (1A) for a blood pressure monitor includes an air bag (10A) having an inflated/deflated space (12) permitting a fluid to come in and out, and a sponge (15A) that is arranged inside the inflated/deflated space (12) to extend substantially continuously from one end (B) to the other end (C) of the air bag (10A) in its winding direction (A). With this configuration, a cuff for a blood pressure monitor permitting the air bag to be inflated uniformly over the entire region when the air is introduced therein is achieved.

## Description

### BACKGROUND OF THE INVENTION

### Field of the Invention

The present invention relates to a cuff for a blood pressure monitor wound around a measurement site of a living body, such as a wrist, an upper arm or the like, for measurement of blood pressure values.

### Description of the Background Art

To measure a blood pressure value, generally, a cuff provided with a fluid bag for pressing an artery located within a living body is wound around the body surface, and arterial pressure pulse waves caused in the artery by inflation/deflation of the fluid bag are detected to measure the blood pressure value. Here, the cuff refers to a band-shaped structure having a bladder, which can be wound around a part of a living body, for use in measurement of arterial pressure of an upper limb, a lower limb or the like by introducing fluid such as gas or liquid into the bladder. Thus, the cuff represents the concept including the fluid bag as well as members for winding the fluid bag around the living body. Particularly, the cuff wound around and fitted on an arm is also called an arm band or a manchette.

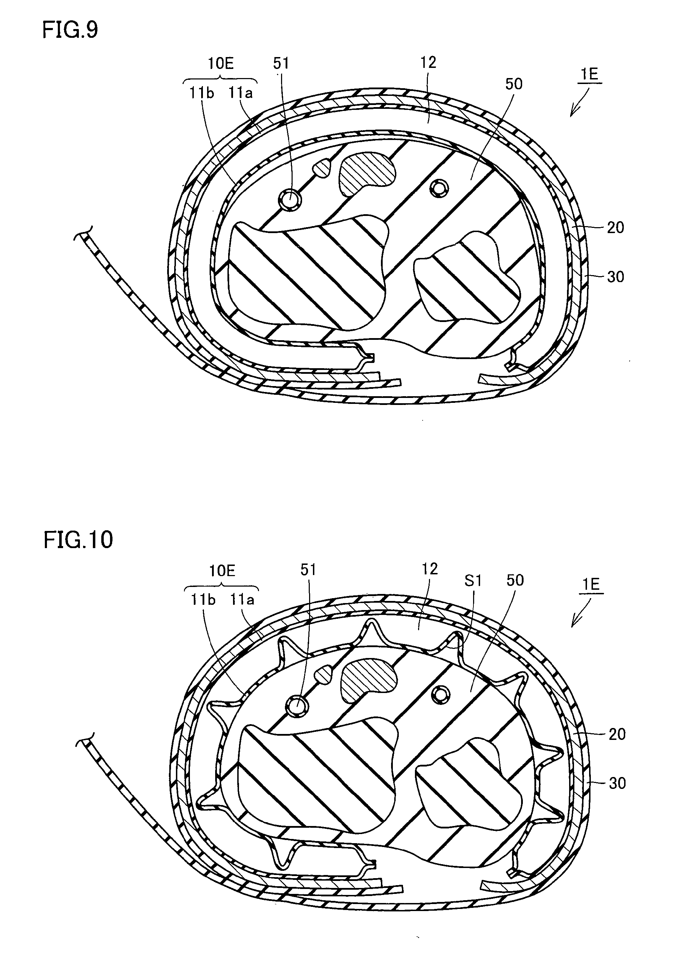

Fig. 9 is a schematic cross sectional view showing a structure of a conventional cuff for a blood pressure monitor, illustrating the state where the cuff is wound around a wrist that is the measurement site. As shown in Fig. 9, the cuff 1E for a blood pressure monitor of the conventional example includes, among others, an air bag 10E that is a fluid bag, a curled elastic member 20 that is an elastic member located on the outside of air bag 10E and wound in an approximately cylindrical shape and changeable in size in a radial direction, and a fastening band 30 for securing the cuff to the living body. Air bag 10E is formed by laying one on another two resin sheets 11a and 11b of an approximately rectangular shape in two dimensions, and by melting and bonding the four sides thereof. Air bag 10E has an inflated/deflated space 12 therein, which is inflated/deflated as the air comes in and out.

As shown in Fig. 9, in the state where cuff 1E is wound around the measurement site of wrist 50, cuff 1E is secured to the wrist in an immovable manner by means of a securing member (not shown) such as a velcro fastener provided at fastening band 30. As such, air bag 10E is wound and positioned between wrist 50 and curled elastic member 20 that is arranged inside fastening band 30.

Fig. 10 is a schematic cross sectional view showing the fitted state of the cuff for a blood pressure monitor in Fig. 9 at the time of measurement of the blood pressure values. As shown in Fig. 10, in the fitted state of the cuff shown in Fig. 9, the pressurized air is introduced into air bag 10E, to make it inflated between curled elastic member 20 and wrist 50. With this inflation of air bag 10E, an artery 51 located under the skin of wrist 50 is pressed by air bag 10E, to enable measurement of the blood pressure values.

For accurate measurement of the blood pressure values, it is necessary for air bag 10E to be inflated uniformly over the entire region when the pressurized air is introduced therein. The problem at this time is occurrence of wrinkles S1 at the surface of air bag 10E inflated by the pressurized air.

Wrinkles S1 are caused when air bag 10E is inflated. Of the resin sheets constituting air bag 10E, resin sheet 11b located inside is decreased in diameter in accordance with inflation of air bag 10E, and the redundant part that cannot escape anywhere gathers locally. Wrinkles S1 occur mostly in the direction crossing the winding direction of cuff 1E around the living body. Occurrence of wrinkles S1 leads to degradation of avascularization performance by air bag 10E, disadvantageously decreasing the accuracy in measurement of blood pressure values. Particularly, in the case where large wrinkles S 1 occur in the proximity of artery 51, as shown in Fig. 10, air bag 10E cannot sufficiently press artery 51 located beneath the skin, causing considerable errors in the blood pressure values measured.

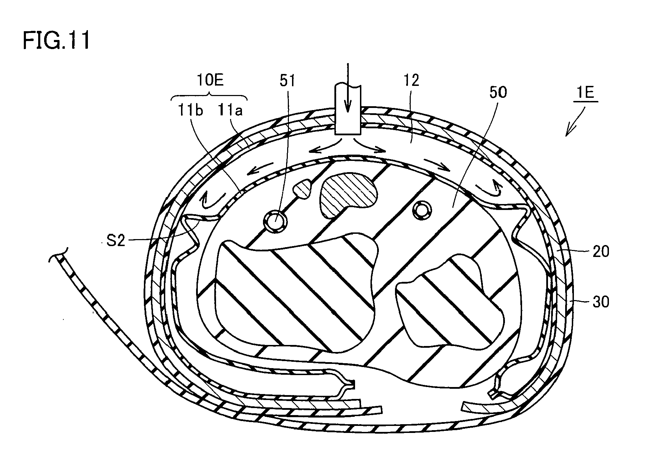

Fig. 11 is a schematic cross sectional view illustrating the problem in the case where wrinkles S 1 occurred are larger and deeper, and further increase in size to cause bending S2 of air bag 10E. As shown in Fig. 11, when bigger and deeper wrinkles S1 occur and increase in size to cause bending S2 of air bag 10E, and bending S2 extends from one end to the other end of air bag 10E in the width direction of cuff 1E (i.e., in the direction approximately parallel to artery 51 in the fitted state of the cuff), then this bending S2 would block the flow of the air (shown by arrows in Fig. 11) being introduced into air bag 10E. In this case, it will not be possible to sufficiently inflate air bag 10E over the entire region, and thus, the blood pressure values measured will include considerable errors.

Japanese Patent Laying-Open Nos. 62-072315 and 2003-038451 disclose techniques for preventing the problems caused by occurrence of wrinkles. According to the technique disclosed in Japanese Patent Laying-Open No. 62-072315, to prevent blood stasis due to wrinkles caused in the fluid bag, a plurality of joint potions for joining one surface on the inner peripheral side and the other surface on the outer peripheral side of the fluid bag are arranged in the longitudinal direction of the fluid bag, so as to intentionally localize occurrence of wrinkles in the air bag in prescribed locations. According to the technique disclosed in Japanese Patent Laying-Open No. 2003-038451, to prevent decrease of accuracy in measurement because of degradation of avascularization performance due to occurrence of wrinkles, cushion members are provided inside the air bag intermittently such that the air bag is maintained in the inflated state before the pressurized air is introduced therein. This localizes the wrinkles caused in the air bag between the cushion members provided intermittently, to thereby prevent occurrence of wrinkles or bending in the portions provided with the cushion members.

With the above-described techniques, however, wrinkles are caused to occur locally in prescribed positions of the air bag, which may rather increase the probability of occurrence of bending in the relevant positions. If such bending occurs in the air bag, the above-described flow of the air in the inflated/deflated space will be blocked, making it difficult to uniformly inflate the air bag over the entire region. In such a case, it is not possible to uniformly and stably press the measurement site, thereby causing measurement errors.

Further, Japanese Patent Laying-Open No. 2004-159967 discloses a technique to prevent bending of the air bag. According to the technique disclosed in Japanese Patent Laying-Open No. 2004-159967, a double-cuff for measuring blood pressure is provided with an air bag for avascularization and an air bag for detecting pulse waves. A core member harder than the air bag for detecting pulse waves is arranged inside the air bag for detecting pulse waves, to prevent bending of the air bag for detecting pulse waves during the fitting operation.

This technique, however, is directed to prevent bending of a small-sized air bag for detecting pulse waves that is arranged only in a specific part between the air bag for avascularization and the measurement site of the living body in the cuff-fitted state. The technique is not for preventing bending of a large-sized air bag for avascularization that is fitted to surround the measurement site. Accordingly, the above-described technique is effective only when the air bag for pressing the living body is formed of separate bags for avascularization and for detection of pulse waves. It does not function effectively for the cuff configured with one air bag that is used both for avascularization and for detection of pulse waves. In other words, in the case of a cuff configured to use one air bag for both avascularization and pulse wave detection, it is necessary to wind the cuff around the living body such that the air bag surrounds the living body in the circumferential direction including the measurement site. Thus, provision of a hard core member inside the air bag would considerably adversely affect the fitting of the cuff.

Further, although the above-described technique may prevent bending of a small-sized air bag for detecting pulse waves, it would not be able to suppress occurrence of large wrinkles or bending of a large-sized air bag for avascularization caused when the pressurized air is introduced therein. Thus, in the case where big wrinkles or bending occur locally in the air bag for avascularization, it is not possible to uniformly press the measurement site over the entire region, resulting in degradation of avascularization performance. Accordingly, the problem of degradation of accuracy in measurement of the blood pressure values is yet to be solved.

### SUMMARY OF THE INVENTION

An object of the present invention is to provide a cuff for a blood pressure monitor permitting a fluid bag to be inflated uniformly through the entire region when the pressurized air is introduced therein.

A cuff for a blood pressure monitor according to the present invention is wound around a living body, and includes: a fluid bag having an inflated/deflated space permitting a fluid to come in and out; and an air-permeable member arranged inside the inflated/deflated space and extending substantially continuously from one end to the other end of the fluid bag in a winding direction thereof.

With this configuration, wrinkles of the fluid bag, caused when the pressurized air is introduced therein, come into contact with the air-permeable member arranged inside the inflated/deflated space. This prevents further increase in size of the wrinkles, and thus, the wrinkles are distributed over the entire region of the fluid bag. In addition, with provision of the air-permeable member that is a porous member, an air passage through which the fluid flows from one end to the other end of the fluid bag in its winding direction is secured, so that the fluid bag is inflated uniformly over the entire region. Accordingly, stable avascularization performance is achieved, and accurate and stable measurement of the blood pressure values is ensured. Herein, the winding direction of the fluid bag with respect to the living body refers to the direction in which the fluid bag wound to surround the living body extends in the fitted state of the cuff. It coincides with the circumferential direction of the cuff wound around the living body in the fitted state.

Preferably, in the cuff for a blood pressure monitor of the present invention, the air-permeable member is placed substantially uniformly through the entire region within the inflated/deflated space.

Thus, by arranging the air-permeable member uniformly through the entire region of the inflated/deflated space, it is possible to uniformly inflate the fluid bag over the entire region more reliably.

In the cuff for a blood pressure monitor of the present invention, the air-permeable member may have an approximately ladder shape in two dimensions.

Even in the case where the air-permeable member contained in the fluid bag is formed in a ladder shape in two dimensions, it is possible to uniformly inflate the fluid bag over the entire region in the winding direction of the fluid bag.

Preferably, in the cuff for a blood pressure monitor of the present invention, the fluid bag is formed by laying one on another two resin sheets of an approximately rectangular shape in two dimensions, and by melting and bonding four sides thereof. In this case, the air-permeable member is preferably placed to extend between a pair of opposite bonded portions of the fluid bag.

With this configuration, it is readily possible to arrange the air-permeable member inside the fluid bag, and accordingly, the cuff for a blood pressure monitor having the above-described configuration can be fabricated with ease.

Preferably, in the cuff for a blood pressure monitor of the present invention, the air-permeable member is a sponge formed by continuous foaming.

Thus, by using the sponge formed by continuous foaming as the air-permeable member, the cuff for a blood pressure monitor having the above-described configuration can be fabricated in a simple and inexpensive manner.

According to the present invention, it is possible to uniformly inflate the fluid bag over the entire region when the pressurized air is introduced therein. As such, stable avascularization performance is achieved at the time of measurement, and accordingly, accurate and stable measurement of the blood pressure values is enabled.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic cross sectional view showing a structure of a cuff for a blood pressure monitor according to an embodiment of the present invention, illustrating the state where the cuff for a blood pressure monitor is wound around a wrist that is a measurement site.
Fig. 2 is a schematic top plan view showing a structure of an air bag of the cuff for a blood pressure monitor according to the embodiment of the present invention.
Figs. 3 and 4 are schematic cross sectional views of the air bag shown in Fig. 2, taken along the line III-III and the line IV-IV, respectively, in Fig. 2.
Fig. 5 is a schematic cross sectional view showing the state where the cuff for a blood pressure monitor shown in Fig. 1 is wound for measurement of the blood pressure values.
Figs. 6-8 are schematic top plan views showing modifications of the cuff for a blood pressure monitor of the embodiment of the present invention.
Fig. 9 is a schematic cross sectional view showing a structure of a conventional cuff for a blood pressure monitor, illustrating the state where the cuff for a blood pressure monitor is wound around a wrist that is the measurement site.
Fig. 10 is a schematic cross sectional view of the conventional cuff for a blood pressure monitor shown in Fig. 9 in the fitted state at the time of measurement.
Fig. 11 is a schematic cross sectional view of the conventional cuff for a blood pressure monitor shown in Fig. 9, showing the case where wrinkles occurred in the air bag have increased in size and depth to cause bending.

### DESCRIPTION OF THE PREFERRED EMBODIMENTS

Hereinafter, an embodiment of the present invention will be described with reference to the drawings. In the following embodiment, a cuff for use in a wrist blood pressure monitor using the wrist as the measurement site for measurement of blood pressure values will be explained by way of example.

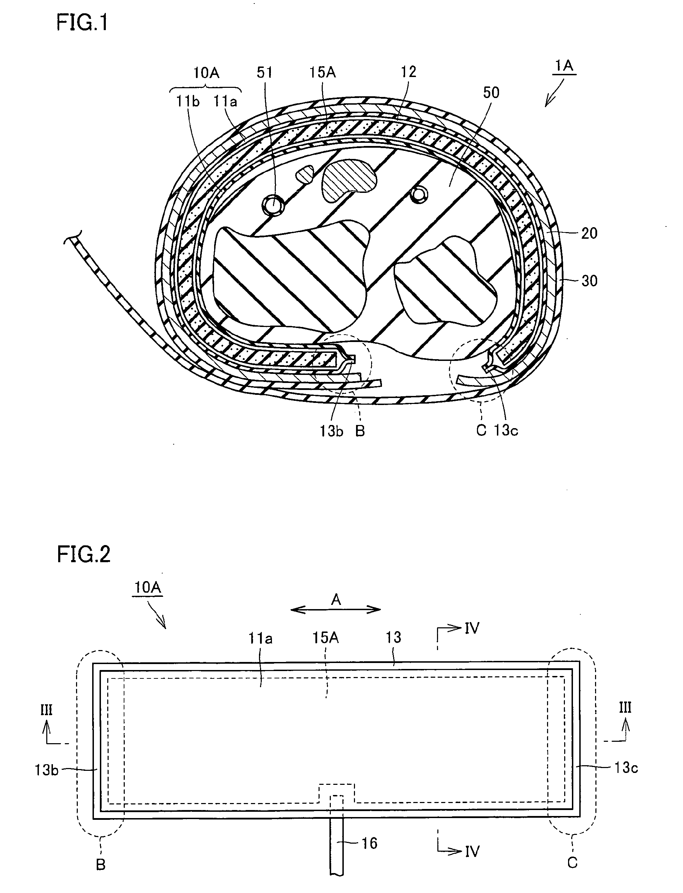

Fig. 1 is a schematic cross sectional view of a cuff for a blood pressure monitor according to an embodiment of the present invention, showing the state where the cuff is wound around a wrist. As shown in Fig. 1, the cuff 1A for a blood pressure monitor of the present embodiment includes, among others, an air bag 10A that is a fluid bag, a curled elastic member 20 that is an elastic member located on the outside of air bag 10A and wound in an approximately cylindrical shape radially changeable in size, and a fastening band 30 for securing cuff 1A to the living body. Air bag 10A has its outer peripheral surface fixed to the inner peripheral surface of curled elastic member 20. The outer peripheral surface of curled elastic member 20 is fixed to the inner peripheral surface of fastening band 30.

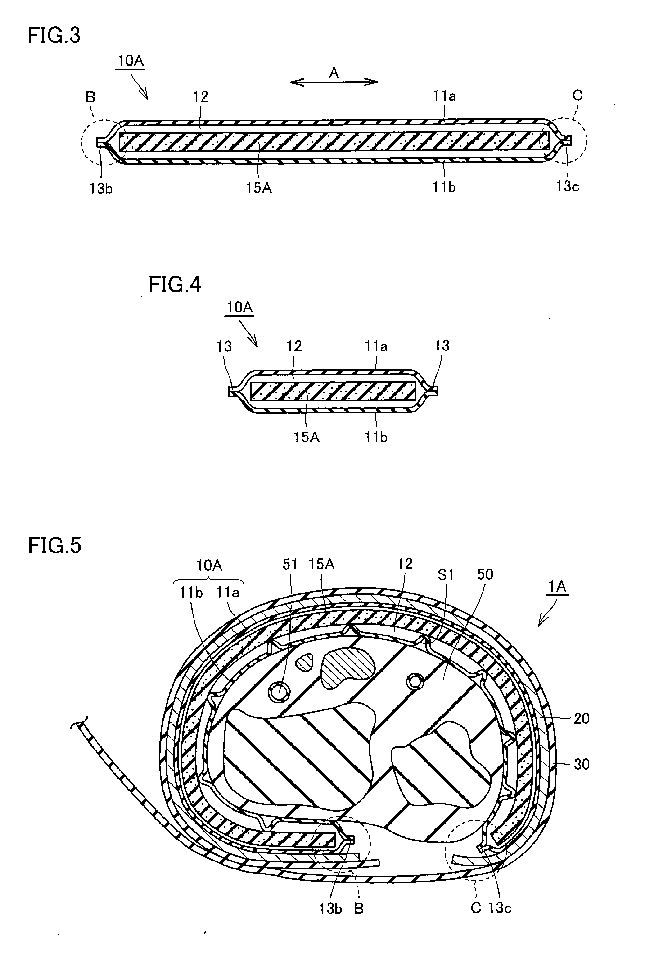

Fig. 2 is a schematic top plan view showing the air bag in Fig. 1 in a spread state. Fig. 3 is a schematic cross sectional view of the air bag in Fig. 2, taken along the line III-III therein, and Fig. 4 is a schematic cross sectional view of the air bag of Fig. 2, taken along the line IV-IV therein. As shown in Figs. 2-4, the air bag 10A of cuff 1A for a blood pressure monitor of the present embodiment is formed by laying one on another two resin sheets 11a and 11b of an approximately rectangular shape in two dimensions and by melting and bonding four sides thereof. As such, air bag 10A has a bonded portion 13 along the rim on the four sides.

As shown in Fig. 2, resin sheets 11a and 11b forming air bag 10A are elongated in the winding direction of cuff 1A on the wrist, which is shown by an arrow A in the figure. Herein, the winding direction on or around the wrist refers to the direction in which air bag 10A wound around the wrist extends in the fitted state of the cuff. Thus, it corresponds to the circumferential direction of cuff 1A applied to surround the wrist in the fitted state. Normally, cuff 1A is formed to be elongated in the winding direction around the wrist, and thus, the longitudinal direction of air bag 10A coincides with the winding direction around the wrist. In the spread state of air bag 10A, this winding direction around the wrist corresponds to the direction shown by an arrow A in Figs. 2 and 3.

An inflated/deflated space 12 that is a hollow space is located inside air bag 10A formed by melting and bonding two resin sheets 11a and 11b together as described above. As shown in Fig. 2, a rubber tube 16 is connected to inflated/deflated space 12 on one end, and the other end of rubber tube 16 is connected to an air system such as a pressurizing pump provided in the main unit of the blood pressure monitor (not shown). With the function of the air system, the air that is a fluid enters and leaves the inflated/deflated space 12 that is the internal space of air bag 10A. Particularly, when the compressed air is introduced into air bag 10A, inflated/deflated space 12 is inflated, causing air bag 10A to be deformed to increase its thickness in the radial direction.

For resin sheets 11a and 11b, any material can be used as long as it is highly stretchable and suppresses leakage of the air from inflated/deflated space 12 after melting and bonding. From these standpoints, optimal materials for resin sheets 11a and 11b may include, e.g., copolymer of ethylene-vinyl acetate (EVA), soft polyvinyl chloride (PVC), polyurethane (PU), crude rubber, and the like.

As shown in Figs. 2-4, inflated/deflated space 12 formed inside air bag 10A is provided with a sponge 15A that is an air-permeable member. This sponge 15A is formed by continuous foaming, and has a huge number of pores through which the air can pass. That is, sponge 15A is formed of a porous member having air permeability. Optimal materials for sponge 15A may include, e.g., urethane and Neoprene (registered trademark).

Sponge 15A has an approximately rectangular shape in two dimensions, and is arranged approximately uniformly through the entire region of inflated/deflated space 12. That is, as shown in Figs. 2 and 3, sponge 15A has an approximately rectangular shape in two dimensions, and is arranged inside inflated/deflated space 12 to extend substantially continuously from one end B to the other end C in the winding direction of air bag 10A, which is shown by an arrow A in the figure. Sponge 15A is positioned to extend between a pair of bonded portions 13b, 13c opposite to each other in the longitudinal direction (shown by the arrow A in the figure) of air bag 10A.

Hereinafter, the fitted state where the cuff 1A for a blood pressure monitor provided with air bag 10A having the above-described configuration is wound around the wrist will be described. As shown in Fig. 1, in the state where cuff 1A is wound around wrist 50, cuff 1A is secured to wrist 50 in an immovable manner by means of a securing member (not shown) such as a velcro fastener provided on fastening band 30. This permits air bag 10A to be wound and positioned between curled elastic member 20 inside fastening band 30 and wrist 50. In this state, there are no wrinkles noticeable on the surface of air bag 10A, since the compressed air is yet to be introduced therein.

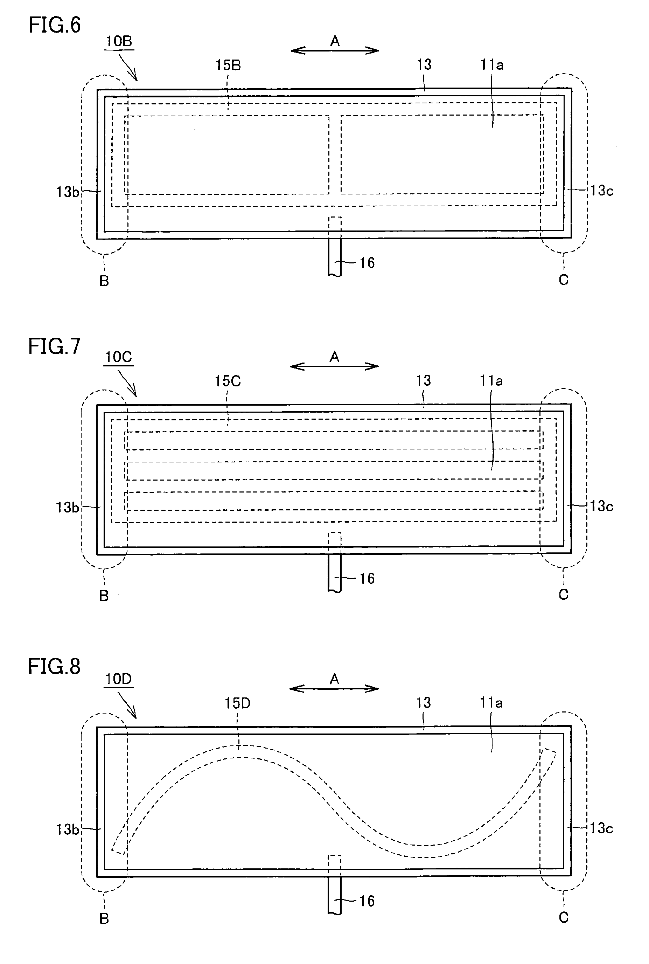

Next, the measurement state where the compressed air is introduced into air bag 10A in the fitted state shown in Fig. 1 will be described. Fig. 5 is a schematic cross sectional view showing the wound state of the cuff for a blood pressure monitor of Fig. 1 at the time of measurement. As shown in Fig. 5, when the compressed air is introduced into air bag 10A in the fitted state in Fig. 1, air bag 10A is inflated between curled elastic member 20 and wrist 50. Correspondingly, of the resin sheets forming air bag 10A, resin sheet 11b located inside comes to suffer wrinkles S1. Wrinkles S 1 are caused as resin sheet 11b is reduced in diameter due to inflation of air bag 10A, and the redundant part that cannot escape anywhere gathers locally.

As described above, in cuff 1A for a blood pressure monitor of the present embodiment, sponge 15A is positioned uniformly through inflated/deflated space 12 of air bag 10A. Thus, wrinkles S1 having occurred on the surface of air bag 10A come to abut against sponge 15A arranged inside inflated/deflated space 12, and are prevented from further increasing in depth. This causes wrinkles to occur at other places in air bag 10A, and as a result, fine and shallow wrinkles are formed at arbitrary locations and thus distributed over the entire region of resin sheet 11b located on the inner peripheral side of air bag 10A. Accordingly, air bag 10A is inflated approximately uniformly over the entire region, and thus, it can uniformly press the surface of wrist 50 over the entire region in the circumferential direction. This enables accurate and stable measurement of the blood pressure values, and occurrence of blood stasis due to the cuff tightened around the wrist is suppressed as well.

Further, in cuff 1A for a blood pressure monitor of the present embodiment, sponge 15A is arranged inside inflated/deflated space 12 of air bag 10A, so that an air passage is secured inside air bag 10A when the compressed air is introduced therein. That is, provision of sponge 15A having a prescribed thickness can prevent resin sheets 11a and 11b forming air bag 10A from coming into close contact with each other in the positions where wrinkles S1 occur. Further, the air permeability of sponge 15A can prevent blockage of inflated/deflated space 12 at the positions suffering wrinkles S1. Accordingly, uneven inflation of the air bag due to bending of the air bag that would occur in a conventional cuff for a blood pressure monitor is prevented, and as a result, stable avascularization performance can be obtained during avascularization. This ensures accurate and stable measurement of the blood pressure values.

Furthermore, air bag 10A containing sponge 15A therein can readily be fabricated by melting and bonding resin sheets 11a and 11b together while sealing sponge 15A therebetween. Thus, a cuff for a blood pressure monitor having stable avascularization performance can be fabricated in a simple and inexpensive manner.

In the embodiment of the present invention described above, cuff 1A for a blood pressure monitor having air bag 10A provided with sponge 15A of an approximately rectangular shape in two dimensions positioned uniformly through the entire region of inflated/deflated space 12 has been explained by way of example. However, the shapes and structures of the sponge identified as the air-permeable member and of the air bag identified as the fluid bag are not restricted thereto. For example, the sponge may be in an approximately ladder shape in two dimensions, as in the cases of sponges 15B and 15C shown in Figs. 6 and 7, respectively. Alternatively, the sponge may have an approximately S shape in two dimensions, as in the case of a sponge 15D shown in Fig. 8. As such, the air-permeable member may have any shape, as long as it is located inside the inflated/deflated space and continuously extends from one end to the other end of the air bag in the winding direction. Further, the air bag does not need to have an approximately rectangular shape in two dimensions, but may have an elliptic shape, for example. The air bag does not necessarily have to be formed of two resin sheets laid one on another. One resin sheet may be folded back to form the air bag, for example.

In the embodiment described above, the case of using a sponge made by continuous foaming as the air-permeable member has been explained by way of example. However, any member may be utilized as long as it has air permeability. For example, fibrous materials such as felt, woven cloth, non-woven cloth and the like may be employed.

In the embodiment described above, the case of applying the present invention to a cuff for use in a wrist blood pressure monitor assuming a wrist as the measurement site has been explained by way of example. The present invention is also applicable to a cuff for a blood pressure monitor of any type, such as the one fitted to an upper arm, the one fitted to a finger, and the like.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A cuff for a blood pressure monitor wound around a living body, comprising:
a fluid bag (10A-10D) having an inflated/deflated space (12) permitting a fluid to come in and out; and
an air-permeable member (15A-15D) arranged inside said inflated/deflated space (12) and extending substantially continuously from one end (B) to the other end (C) of said fluid bag (10A-10D) in a winding direction (A) thereof.

2. The cuff for a blood pressure monitor according to claim 1, wherein said air-permeable member (15A-15D) is placed substantially uniformly through the entire region within said inflated/deflated space (12).

3. The cuff for a blood pressure monitor according to claim 1, wherein said air-permeable member (15B, 15C) has an approximately ladder shape in two dimensions.

4. The cuff for a blood pressure monitor according to claim 1, wherein
said fluid bag (10A-10D) is formed by laying one on another two resin sheets (11a, 11b) of an approximately rectangular shape in two dimensions, and by melting and bonding four sides thereof, and
said air-permeable member (15A-15D) is placed to extend between a pair of opposite bonded portions (13b, 13c) of said fluid bag (10A-10D).

5. The cuff for a blood pressure monitor according to claim 1, wherein said air-permeable member (15A-15D) is a sponge formed by continuous foaming.
